(19) Europäisches Patentamt
European Patent Office
Office européen des brevets

(11) Veröffentlichungsnummer: **0 199 238**
**A1**

(12) **EUROPÄISCHE PATENTANMELDUNG**

(21) Anmeldenummer: 86105093.8

(22) Anmeldetag: 14.04.86

(51) Int. Cl.⁴: C 07 D 211/90
A 61 K 31/44

(30) Priorität: 25.04.85 DE 3514865

(43) Veröffentlichungstag der Anmeldung: 29.10.86 Patentblatt 86/44

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Wehinger, Egbert, Dr.
Gellertweg 33
D-5600 Wuppertal 1(DE)

(72) Erfinder: Scherling, Dietrich, Dr.
Funkstrasse 20a
D-5600 Wuppertal 1(DE)

(72) Erfinder: Rämsch, Klaus-Dieter, Dr.
Scheidstrasse 141
D-5600 Wuppertal 21(DE)

(72) Erfinder: Knorr, Andreas, Dr.
Pahlkestrasse 15
D-5600 Wuppertal 1(DE)

(72) Erfinder: Kazda, Stanislav, Dr.
Gellertweg 18
D-5600 Wuppertal 1(DE)

(54) **1,4-Dihydropyridin-Derivate, Verfahren zu ihrer Herstellung und ihre Verwendung in Arzneimitteln.**

(57) Die Erfindung betrifft neue 1,4-Dihydropyridin-Derivate der allgemeinen Formel I

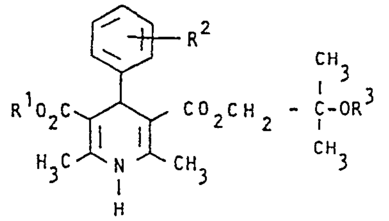

(I)

in welcher $R^1$, $R^2$ und $R^3$ die in der Beschreibung angegebene Bedeutung haben, mehrere Verfahren zu ihrer Herstellung und ihre Verwendung in Arzneimitteln mit kreislaufbeeinflussender Wirkung.

EP 0 199 238 A1

Croydon Printing Company Ltd

BAYER AKTIENGESELLSCHAFT
Konzernverwaltung RP
Patentabteilung

5090 Leverkusen, Bayerwerk

KS/by-c
Ia(Pha)

1,4-Dihydropyridin-Derivate, Verfahren zu ihrer Herstellung und ihre Verwendung in Arzneimitteln

Die Erfindung betrifft neue 1,4-Dihydropyridin-Derivate, mehrere Verfahren zu ihrer Herstellung und ihre Verwendung in Arzneimitteln mit kreislaufbeeinflussender Wirkung.

Es ist bekannt, daß man 1,4-Dihydro-2,6-dimethyl-4-phenyl-pyridin-3,5-dicarbonsäurediethylester erhält, wenn man Benzylidenacetessigsäureethylester mit β-Amino-crotonsäureethylester oder Acetessigsäureethylester und Ammoniak umsetzt [Knoevenagel, Ber. dtsch. chem. Ges. 31, 743 (1898)].

Weiterhin ist bekannt, daß bestimmte 1,4-Dihydropyridine interessante pharmakologische Eigenschaften aufweisen [vgl. F. Bossert, W. Vater, Naturwissenschaften 58, 578 (1971); DOS 21 17 571].

Gegenstand der Erfindung sind neue 1,4-Dihydropyridin-Derivate der allgemeinen Formel I,

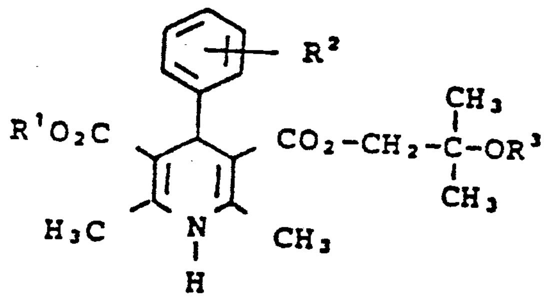

(I)

in welcher

$R^1$ für einen geradkettigen, verzweigten oder cyclischen, gesättigten oder ungesättigten Kohlenwasserstoffrest mit bis zu 6 C-Atomen steht,

$R^2$ für 2-Nitro oder 3-Nitro steht, und

$R^3$ für Wasserstoff oder Acetyl steht.

Es wurde gefunden, daß die Verbindungen der Formel (I) starke gefäßdilatierende Eigenschaften aufweisen und somit als Wirkstoffe in Arzneimitteln eingesetzt werden können.

Von besonderer Bedeutung sind Verbindungen der Formel (I) in welcher $R^1$ für Alkyl mit 1 bis 4 C-Atomen steht.

Außerdem wurde gefunden, daß man die Verbindungen der allgemeinen Formel Ia,

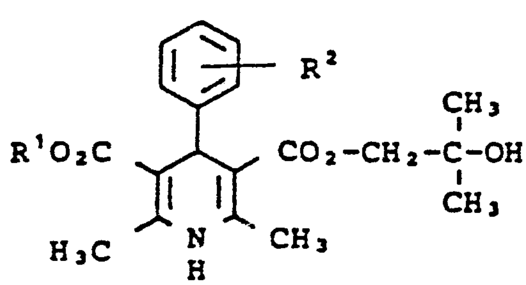

(Ia)

in welcher

$R^1$ und $R^2$ die oben angegebene Bedeutung haben,

erhält, wenn man

(A) 2-Benzyliden-acetessigester der allgemeinen Formel II,

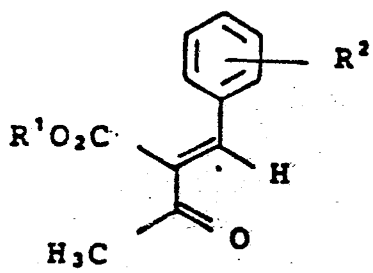

(II)

in welcher

$R^1$ und $R^2$ die oben angegebene Bedeutung haben,

mit 2-Aminocrotonsäure-(2-acetoxy-2-methyl-propyl)-ester der Formel III

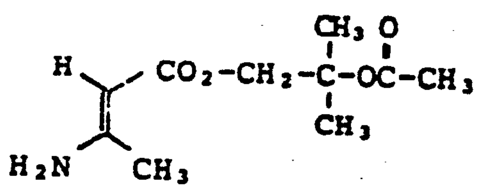

(III)

gegebenenfalls in Gegenwart von Wasser und/oder inerten organischen Lösungsmittel umsetzt,

oder

(B) 2-Benzyliden-acetessigester der allgemeinen Formel II mit Ammoniak und Acetessigsäure-(2-acetoxy-2-methyl-propyl)ester der Formel IV,

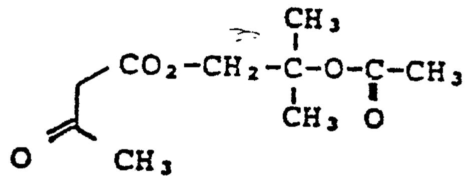

(IV)

gegebenenfalls in Gegenwart von Wasser und/oder inerten organischen Lösungsmitteln umsetzt,

oder

(C) Enamine der allgemeinen Formel V,

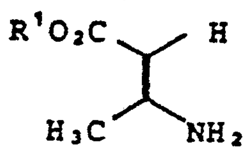

(V)

in welcher

$R^1$ die oben angegebene Bedeutung hat,

mit 2-Benzyliden-acetessigestern der allgemeinen Formel VI,

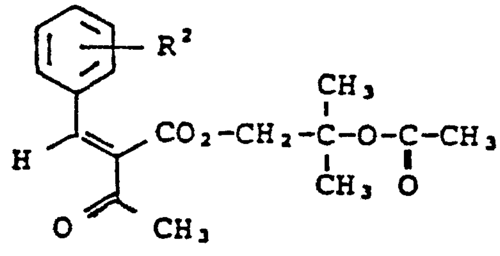

(VI)

in welcher

$R^2$ die oben angegebene Bedeutung hat,

gegebenenfalls in Gegenwart von Wasser und/oder inerten organischen Lösungsmitteln umsetzt,

oder

(D) 2-Benzyliden-acetessigester der allgemeinen Formel VI mit Ammoniak und Acetessigestern der allgemeinen Formel VII,

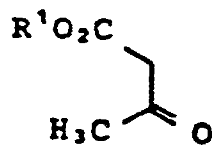

(VII)

in welcher

$R^1$ die oben angegebene Bedeutung hat,

gegebenenfalls in Gegenwart von Wasser und/oder inerten organischen Lösungsmitteln umsetzt,

oder

(E) Enamine der allgemeinen Formel V und Acetessigsäure-(2-acetoxy-2-methyl-propyl)ester der Formel IV mit Aldehyden der allgemeinen Formel VIII,

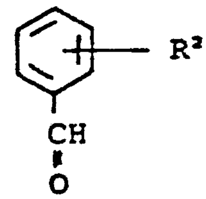

(VIII)

in welcher

$R^2$ die oben angegebene Bedeutung hat,

gegebenenfalls in Gegenwart von Wasser und/oder inerten organischen Lösungsmitteln umsetzt,

oder

(F) 2-Aminocrotonsäure-(2-acetoxy-2-methyl)propylester der Formel III mit Acetessigestern der allgemeinen Formel VII und Aldehyden der allgemeinen Formel VIII gegebenenfalls in Gegenwart von Wasser und/oder inerten organischen Lösungsmitteln umsetzt,

und anschließend die so erhaltenen 1,4-Dihydropyridine der allgemeinen Formel Ib,

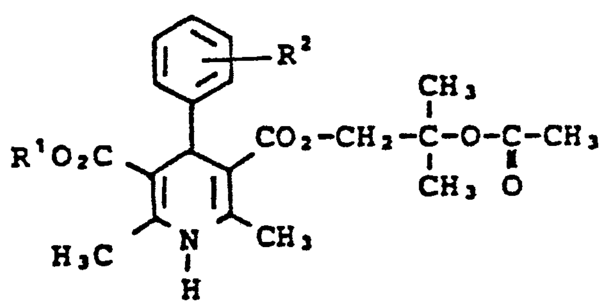

(Ib)

in welcher

$R^1$ und $R^2$ die oben angegebene Bedeutung haben,

gegebenenfalls in Gegenwart von Säuren oder Basen zu den erfindungsgemäßen Verbindungen der allgemeinen Formel Ia, unter solvolytischen Bedingungen umsetzt.

Überraschenderweise zeigen die erfindungsgemäßen Stoffe starke vasodilatierende Eigenschaften und können somit in Arzneimitteln eingesetzt werden. Sie stellen somit eine Bereicherung der Pharmazie dar.

Die erfindungsgemäßen Verbindungen sind chiral und können in stereoisomeren Formen existieren, die sich wie Bild und Spiegelbild verhalten. Diese können ihrerseits wieder in verschiedenen Konformationen auftreten. Sowohl die Racemformen als auch die Antipoden sind Gegenstand der vorliegenden Erfindung.

Je nach Art der verwendeten Ausgangsstoffe kann die Synthese der erfindungsgemäßen Verbindungen beispielhaft durch folgende Formelschemata wiedergegeben werden:

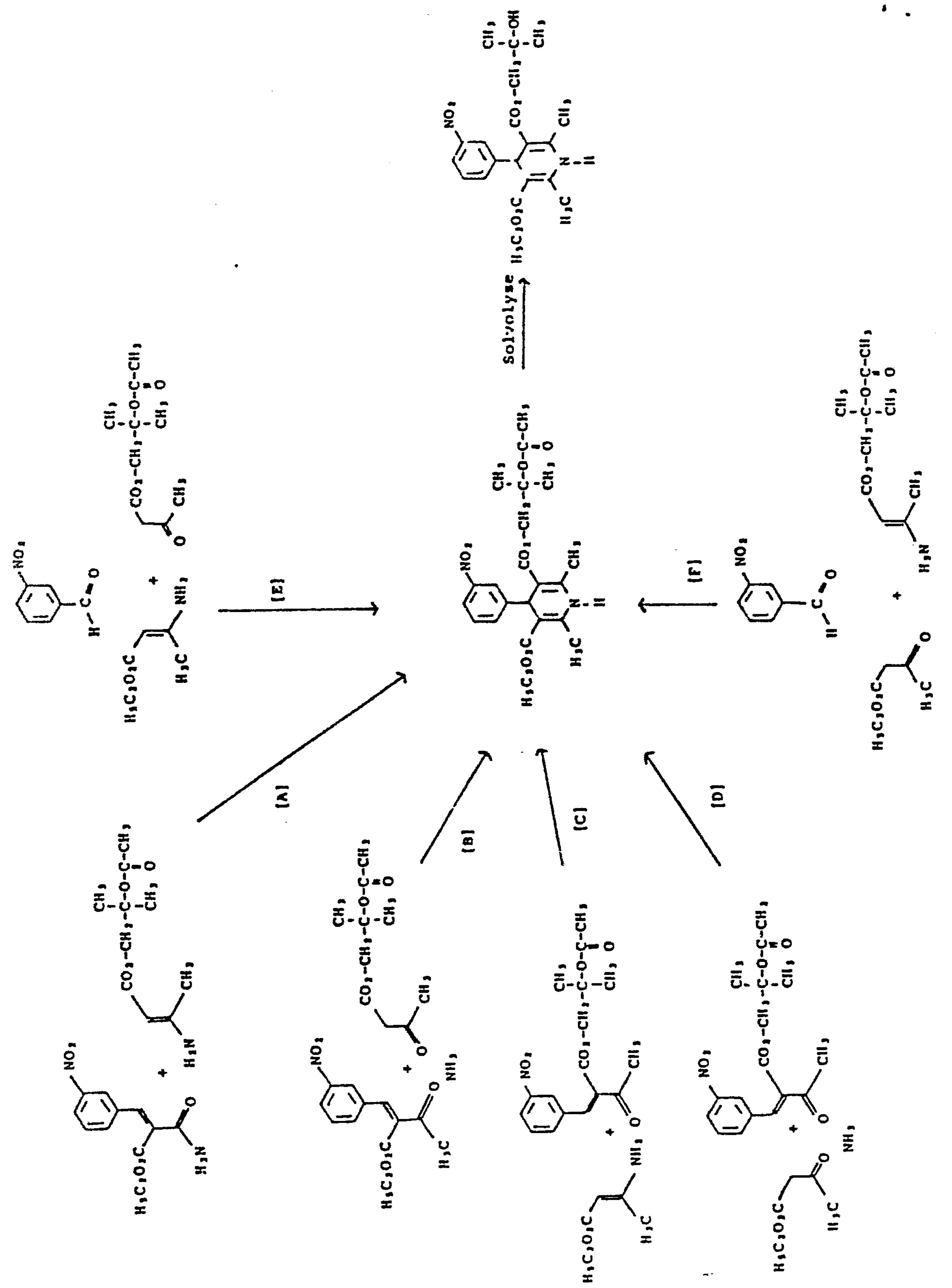

Solvolyse
[A]
[B]
[C]
[D]
[E]
[F]

Die Verbindungen der Formeln II bis VIII sind bekannt oder können nach literaturbekannten Methoden hergestellt werden [vgl. G. Jones, "The Knoevenagel Condensation" in Organic Reactions Vol. XV, 204 ff. (1967); S.A. Glickman, A.C. Cope, J. Am. Chem. Soc. 67, 1017 (1945); D. Borrmann, "Umsetzung von Diketen mit Alkoholen, Phenolen und Mercaptanen" in Houben-Weyl, Methoden der organischen Chemie, Band VII/4, 230 ff. (1968); E. Mosettig, Organic Reactions Vol. III, 218 ff. (1954)].

Die Verbindungen der allgemeinen Formel Ib ($R^3$ = Acetyl) sind neu und ebenfalls Gegenstand der Erfindung. Sie können in der angegebenen Weise nach Verfahren A-F hergestellt werden.

Als Lösungsmittel für die Verfahren A-F kommen Wasser und alle inerten organischen Lösungsmittel in Frage. Hierzu gehören vorzugsweise Alkohole wie beispielsweise Methanol, Ethanol, n-Propanol oder iso-Propanol, Ether wie beispielsweise Diethylether, Tetrahydrofuran oder Dioxan, Pyridin, Acetonitril, Dimethylformamid, Dimethylsulfoxid oder Hexamethylphosphorsäuretriamid.

Die Reaktionstemperaturen können in einem größeren Bereich variiert werden. Im allgemeinen arbeitet man in einem Bereich von 20°C bis 180°C, vorzugsweise von 50°C bis 120°C. Insbesondere arbeitet man bei der Siedetemperatur des jeweiligen Lösungsmittels.

Die Umsetzung kann bei Normaldruck oder bei erhöhtem Druck durchgeführt werden. Im allgemeinen arbeitet man bei Normaldruck.

Bei der Durchführung der Verfahren A-F werden die an der Reaktion beteiligten Stoffe jeweils etwa in molaren Mengen eingesetzt. Das Ammoniak wird zweckmäßig im Überschuß zugegeben, bevorzugt in 1,5 bis 2,5 molaren Mengen, bezogen auf jeweils 1 Mol eines anderen Ausgangsstoffes.

Die Überführung der Verbindungen Ib ($R^3$ = Acetyl) in die Verbindungen Ia ($R^3$ = Wasserstoff) erfolgt nach üblichen Methoden der Solvolyse, gegebenenfalls in Anwesenheit von Säuren oder Basen.

Als Solvolysereagentien können in reiner oder gemischter Form entweder Wasser oder Alkohole wie beispielsweise Methanol, Ethanol oder Propanol oder Gemische derselben mit inerten organischen Lösungsmitteln wie Dioxan, Tetrahydrofuran oder Dimethoxyethan, Acetonitril, Dimethylformamid oder Dimethylsulfoxid verwendet werden.

Die Reaktion kann gegebenenfalls mit Säuren oder Basen als Hilfsmittel durchgeführt werden. Als Säuren eignen sich die üblichen anorganischen und organischen Säuren, bevorzugt anorganische Säuren wie beispielsweise Chlor- oder Bromwasserstoff, Schwefelsäure, Phosphorsäure, Perchlorsäure, oder Essigsäure oder Toluolsulfonsäure. Als Basen können die üblichen anorganischen oder organischen Basen verwendet werden, bevorzugt Alkali- oder Erdalkalihydroxide wie beispielsweise Lithium-, Natrium-, Kalium-, Calcium- oder Bariumhydroxid, Alkali- oder Erdalkalicarbonate wie z.B. Natrium-, Kalium- oder Calciumcarbonat, Alkalialkoholate wie beispielsweise Natrium- oder Kaliummethanolat, Natrium- oder Kaliumethanolat, Kalium-tert.-butylat oder Amine wie beispielsweise Ammoniak, Triethylamin oder Pyridin. Bevorzugt wird die Solvolyse mit Alkalihydroxiden oder Alkalialkoholaten durchgeführt.

Die Reaktionstemperaturen können in einem größeren Bereich variiert werden. Im allgemeinen arbeitet man bei Temperaturen von 0°C bis 150°C, bevorzugt von 20°C bis 150°C.

Die Solvolyse kann bei Normaldruck, aber auch bei erhöhtem Druck durchgeführt werden. Im allgemeinen arbeitet man bei Normaldruck.

Die vorstehenden Herstellungsverfahren für die erfindungsgemäßen Stoffe der allgemeinen Formel I sind lediglich zur Verdeutlichung angegeben und nicht auf diese Verfahren beschränkt, sondern jede Modifikation der Verfahren ist in gleicher Weise für die Herstellung der Verbindungen I anwendbar.

Die erfindungsgemäßen Verbindungen können als Arzneimittel eingesetzt werden. Sie bewirken bei enteraler oder parenteraler Anwendung eine starke Vasodilatation und können daher zur Therapie und Prophylaxe von Herz-Kreislauferkrankungen eingesetzt werden.

Im einzelnen konnten im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt. Sie beeinflussen bzw. verändern den Herzstoffwechsel im Sinne der Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren. Die Verbindungen eignen sich daher besonders als Cerebraltherapeutika.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, die an der glatten Muskulatur des Magens, Darmtraktes, des Urogenitaltraktes und des Respirationssystems deutlich werden.

Die pharmakologische Wirkung der erfindungsgemäßen Verbindungen I wurde in folgenden Tests nachgewiesen:

Blutdruckmessung bei wachen Ratten

Meßmethode: In Anlehnung an das von Breuninger beschriebene Verfahren wird der Blutdruck am Schwanz der nicht narkotisierten Ratte unblutig mittels aufblasbarer

Gummimanschette und Infraton-Pulsabnehmer D nach Boucke-Brecht gemessen. Die Tiere befinden sich während des ganzen Versuches, einschließlich einer 2-stündigen Vorperiode, einzeln in einem Wassermantel umgeben, auf ca. 30°C temperierten Kunststoffröhren. Die aufblasbare Gummimanschette sitzt an der Schwanzwurzel, der Pulsabnehmer 2-5 cm distal davon. Zur Erzielung gleichmäßiger Meßresultate ist für die wiederholten Messungen die Meßstelle am Schwanz mit Tusche markiert.
Der gesamte Meßvorgang wird auf einem 2-Kanal-Direktschreiber oder einem Speicher-Oszillografen (Tektronix) registriert, im 1. Kanal die Pulsationen der Schwanzarterie, im 2. Kanal die Impulse eines Druckmarkengebers, der den jeweils in der Manschette herrschenden Druck anzeigt. Gemessen wird bei abfallendem Manschettendruck. Als Meßwert wird der in der Manschette herrschende Druck bei Wiederauftreten der Pulsationen benutzt. Er entspricht dem systolischen Blutdruck im Rattenschwanz.

Versuchsablauf: Für die Versuche werden nur Ratten mit einem systolischen Blutdruck über 150 mm Hg verwendet. Beim größten Teil der Tiere liegen die Blutdruckwerte zwischen 170 und 210 mm Hg, in Einzelfällen werden Werte bis 270 mm Hg erreicht. Zwei Stunden vor Versuchsbeginn werden die Tiere zur Eingewöhnung in die vorgewärmten Kunststoffröhren gesetzt, wo sie bis zum Versuchsende verbleiben, also insgesamt 8 Stunden. Nach Bestimmung des Ausgangsblutdruckes wird den Tieren die zu untersuchende Substanz mittels Schlundsonde oral appliziert. Danach wird der Blutdruck 1, 2, 4 und 6 Stunden, sowie am folgenden Tag 24 Stunden nach der Applikation gemessen. Jeder

Meßwert ist das Mittel aus drei unmittelbar aufeinanderfolgenden Einzelmessungen.
Für jede Dosis werden bei Screening-Versuchen 3 Tiere, bei allen anderen Versuchen 6 bis 12 Tiere zu einer Gruppe zusammengefaßt. Pro Versuchstag läuft eine unbehandelte Gruppe als Kontrolle parallel.

Auswertung der Versuchsergebnisse:
Bei der quantitativen Bestimmung von Dosis-Wirkungskurven wird für jedes Tier die Wirkung als mittlere prozentuale Blutdrucksenkung gegenüber dem Ausgangsblutdruck, gemittelt über den Versuchszeitraum von 6 Stunden, angegeben. Mit diesen Werten können lineare Regressionsgeraden aufgestellt, sowie relative Wirksamkeiten mittels linearer Regressionsanalyse berechnet werden. Die niedrigste Dosis, die noch wenigstens 15 mm Hg Blutdrucksenkung ergibt, wird als Grenzdosis bezeichnet.

Literatur:
Breuninger, H.: Methode zur unblutigen Messung des Blutdruckes an Kleintieren. Arzneimittelforschung 6, 222-225 (1956).

Die Grenzdosis der antihypertensiven Wirkung (SH-Ratte) für die Verbindung des Beispiels 3 beträgt z.B. 1 mg/kg p.o..

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung

inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendung gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von

dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele:

### Beispiel 1

1,4-Dihydro-2,6-dimethyl-4-(2-nitrophenyl)-pyridin-3,5-dicarbonsäure-(2-acetoxy-2-methyl-propyl)-methyl-ester

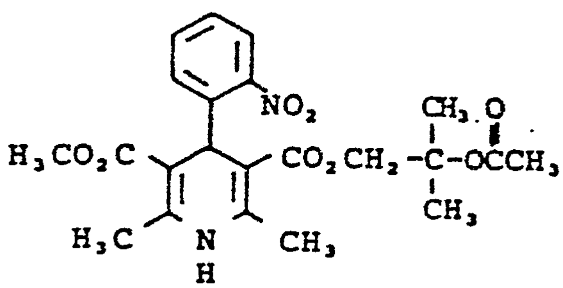

### Verfahrensvariante A

Eine Lösung von 16,9 g (67 mmol) 2-(2-Nitrobenzyliden)-acetessigsäuremethylester und 14,4 g (67 mmol) 3-Aminocrotonsäure-(2-acetoxy-2-methyl-propyl)ester in 50 ml Isopropanol wurde 10 Stunden zum Sieden erhitzt. Anschließend wurde das Lösungsmittel im Vakuum abdestilliert und der ölige Rückstand säulenchromatographisch über Kieselgel mit Trichlormethan/Essigester = 2/1 als Eluierungsmittel gereinigt.
Schmelzpunkt: 113-115°C, Ausbeute: 11,2 g (37 %)

### Verfahrensvariante B

Eine Lösung von 16,9 g (67 mmol) 2-(2-Nitrobenzyliden)-acetessigsäuremethylester, 14,5 g (67 mmol) Acetessig-

säure-(2-acetoxy-2-methyl-propyl)ester und 6,9 g (101 mmol) einer 25 %igen wäßrigen Ammoniaklösung in 70 ml Methanol wurde 14 Stunden unter Rückfluß erhitzt. Das Lösungsmittel wurden unter vermindertem Druck abdestilliert, der ölige Rückstand durch Animpfen einer authentischen Probe zur Kristallisation gebracht, das Rohprodukt aus Ethanol umkristallisiert.
Schmelzpunkt: 113-114°C, Ausbeute: 8,9 g (29,7 %)

Verfahrensvariante C

Eine Lösung von 23,4 g (67 mmol) 2-(2-Nitrobenzyliden)-acetessigsäure-(2-acetoxy-2-methyl-propyl)ester und 7,7 g (67 mmol) 3-Aminocrotonsäuremethylester in 50 ml Ethanol wurde 12 Stunden unter Rückfluß erhitzt. Anschließend wurde das Lösungsmittel im Vakuum abdestilliert und der ölige Rückstand auf einer Kieselgelsäule unter Verwendung von Trichlormethan/Essigester = 2/1 als Eluierungsmittel gereinigt.
Schmelzpunkt: 113-115°C, Ausbeute: 10,2 g (34,1 %)

Verfahrensvariante D

Eine Lösung von 23,4 g (67 mmol) 2-(2-Nitrobenzyliden)-acetessigsäure-(2-acetoxy-2-methyl)propylester, 7,8 g (67 mmol) Acetessigsäuremethylester und 6,9 g (101 mmol) einer 25 %igen wäßrigen Ammoniaklösung in 70 ml Methanol wurde 14 Stunden unter Rückfluß erhitzt. Das Lösungsmittel wurde im Vakuum abdestilliert, der Rückstand säulenchromatographisch unter Verwendung von Kieselgel und Trichlormethan/Essigester = 2/1 gereinigt.
Schmelzpunkt: 112-114°C, Ausbeute: 10 g (33,4 %)

Verfahrensvariante E

Eine Lösung von 10,1 g (67 mmol) 2-Nitrobenzaldehyd, 14,5 g (67 mmol) Acetessigsäure-(2-acetoxy-2-methyl)propylester und 7,7 g (67 mmol) 3-Aminocrotonsäuremethylester in 70 ml Dioxan wurde 15 Stunden unter Rückfluß erhitzt. Das Lösungsmittel wurde unter vermindertem Druck abdestilliert und der Rückstand säulenchromatigraphisch auf Kieselgel mit Trichlormethan/Essigester = 2/1 gereinigt. Schmelzpunkt: 112-114°C, Ausbeute: 8 g (26,7 %)

Verfahrensvariante F

Eine Lösung von 10,1 g (67 mmol) 2-Nitrobenzaldehyd, 7,8 g (67 mmol) Acetessigsäuremethylester und 14,4 g (67 mmol) 3-Aminocrotonsäure-(2-acetoxy-2-methyl-propyl)ester in 70 ml Isopropanol wurde 15 Stunden unter Rückfluß erhitzt. Nach Erkalten der Reaktionsmischung wurde das Lösungsmittel im Vakuum abdestilliert und der Rückstand säulenchromatographisch unter Verwendung von Kieselgel und Trichlormethan/Essigester = 2/1 gereinigt.
Schmelzpunkt: 112-114°C, Ausbeute: 7,5 g (25 %)

Beispiel 2

1,4-Dihydro-2,6-dimethyl-4-(3-nitrophenyl)-pyridin-3,5-dicarbonsäure-(2-acetoxy-2-methyl-propyl)-ethyl-ester

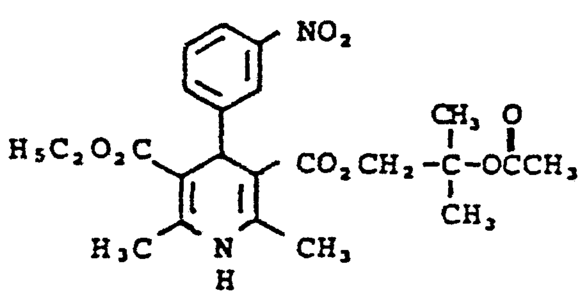

Verfahrensvariante A

Analog Beispiel 1 wurde durch Umsetzung von 2-(3-Nitrobenzyliden)-acetessigsäureethylester mit 3-Aminocrotonsäure-(2-acetoxy-2-methyl-propyl)ester in Isopropanol der 1,4-Dihydro-2,6-dimethyl-4-(3-nitrophenyl)-pyridin-3,5-dicarbonsäure-(2-acetoxy-2-methyl-propyl)-ethyl-ester vom Fp. 113-114°C erhalten. Ausbeute: 33 %

Verfahrensvariante B

Analog Beispiel 1 wurde durch Umsetzung von 2-(3-Nitrobenzyliden)-acetessigsäureethylester mit Acetessigsäure-(2-acetoxy-2-methyl-propyl)ester und Ammoniak in Methanol der 1,4-Dihydro-2,6-dimethyl-4-(3-nitrophenyl)-pyridin-3,5-dicarbonsäure-(2-acetoxy-2-methyl-propyl)-ethyl-ester vom Fp. 112-114°C erhalten.
Ausbeute: 27 %

Verfahrensvariante C

Analog Beispiel 1 wurde durch Umsetzung von 2-(3-Nitrobenzyliden)-acetessigsäure-(2-acetoxy-2-methyl-propyl)-ester und 3-Aminocrotonsäureethylester in Ethanol der 1,4-Dihydro-2,6-dimethyl-4-(3-nitrophenyl)-pyridin-3,5-dicarbonsäure-(2-acetoxy-2-methyl-propyl)-ethyl-ester vom Fp. 113-115°C erhalten.
Ausbeute: 31 %

Verfahrensvariante D

Analog Beispiel 1 wurde durch Umsetzung von 2-(3-Nitrobenzyliden)-acetessigsäure-(2-acetoxy-2-methyl-propyl)-ester mit Acetessigsäureethylester und Ammoniak in Methanol der
1,4-Dihydro-2,6-dimethyl-4-(3-nitrophenyl)-pyridin-3,5-dicarbonsäure-(2-acetoxy-2-methyl-propyl)-ethyl-ester vom Fp. 113-115°C erhalten.
Ausbeute: 28 %

Verfahrensvariante E

Analog Beispiel 1 wurde durch Umsetzung von 3-Nitrobenzaldehyd mit Acetessigsäure-(2-acetoxy-2-methyl-propyl)-ester und 3-Aminocrotonsäureethylester in Ethanol der 1,4-Dihydro-2,6-dimethyl-4-(3-nitrophenyl)-pyridin-3,5-dicarbonsäure-(2-acetoxy-2-methyl-propyl)-ethyl-ester vom Fp.: 111-113°C erhalten. Ausbeute: 23 %

Verfahrensvariante F

Analog Beispiel 1 wurde durch Umsetzung von 3-Nitrobenzaldehyd mit Acetessigsäureethylester und 3-Aminocrotonsäure-(2-acetoxy-2-methyl)propylester in Isopropanol der
1,4-Dihydro-2,6-dimethyl-4-(3-nitrophenyl)-pyridin-3,5-dicarbonsäure-5-methyl-3-(2-acetoxy-2-methyl)propylester vom Fp.: 113-115°C erhalten.
Ausbeute: 25 %

Beispiel 3

1,4-Dihydro-2,6-dimethyl-4-(2-nitrophenyl)-pyridin-3,5-dicarbonsäure-(2-hydroxy-2-methyl-propyl)-methyl-ester

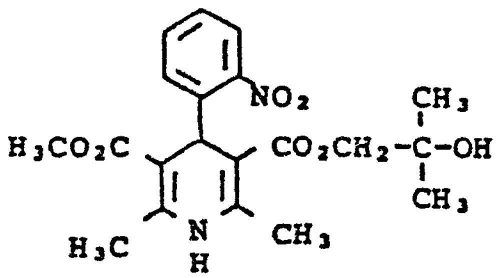

6,5 g (14,5 mmol) 1,4-Dihydro-2,6-dimethyl-4-(2-nitrophenyl)-pyridin-3,5-dicarbonsäure-(2-acetoxy-2-methyl-propyl)-methyl-ester wurden in einer Lösung von 0,63 g (15,8 mmol) Natriumhydroxid in 14 ml Wasser plus 28 ml 1,2-Dimethoxyethan 3 Stunden bei Raumtemperatur gerührt. Anschließend wurde die Lösung in Wasser aufgenommen und mit Dichlormethan extrahiert. Die organische Phase wurde nach Trocknen über Natriumsulfat unter vermindertem Druck eingeengt und der ölige Rückstand über Kieselgel mit Trichlormethan/Essigester = 2/1 als Eluierungsmittel gereinigt.
Schmelzpunkt: 127-129°C, Ausbeute: 3,5 g (60 %)

Beispiel 4

1,4-Dihydro-2,6-dimethyl-4-(3-nitrophenyl)-pyridin-3,5-dicarbonsäure-(2-hydroxy-2-methyl-propyl)-ethyl-ester

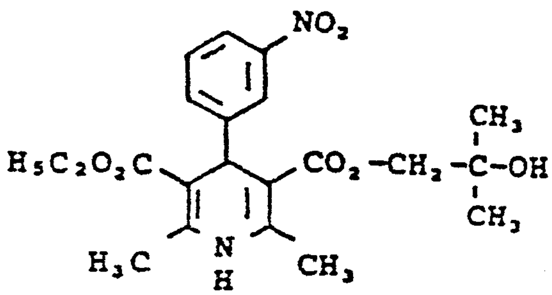

5 g (10,8 mmol) 1,4-Dihydro-2,6-dimethyl-4-(3-nitrophenyl)-pyridin-3,5-dicarbonsäure-(2-acetoxy-2-methylpropyl)-ethyl-ester wurden in einer Lösung von 0,47 g (11,8 mmol) Natriumhydroxid in 11 ml Wasser plus 22 ml 1,2-Dimethoxyethan 3 Stunden bei Raumtemperatur gerührt. Anschließend wurde die Reaktionsmischung mit Wasser verdünnt und mit Dichlormethan extrahiert, die organischen Extrakte über Natriumsulfat getrocknet und im Vakuum eingeengt. Der ölige Rücklstand wurde säulenchromatographisch auf Kieselgel mit Trichlormethan/Essigester = 2/1 gereinigt.
Schmelzpunkt: 154-156°C, Ausbeute: 2,6 g (58 %)

## Patentansprüche

1. 1,4-Dihydropyridin-Derivate der allgemeinen Formel I

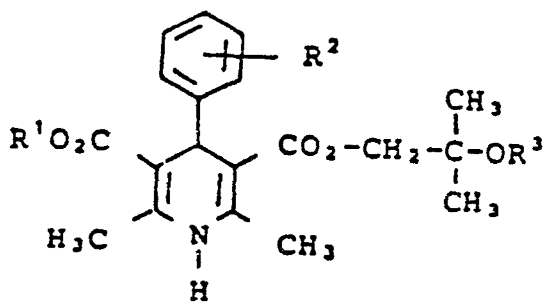

(I)

in welcher

$R^1$ für einen geradkettigen, verzweigten oder cyclischen, gesättigten oder ungesättigten Kohlenwasserstoffrest mit bis zu 6 C-Atomen steht,

$R^2$ für 2-Nitro oder 3-Nitro steht, und

$R^3$ für Wasserstoff oder Acetyl steht.

2. Verbindungen der allgemeinen Formel I gemäß Anspruch 1 in welcher

$R^1$ für Alkyl mit 1 bis 4 C-Atomen steht und

$R^2$ und $R^3$ die in Anspruch 1 angegebene Bedeutung haben.

3. Verfahren zur Herstellung von Verbindungen der allgemeinen Formel I

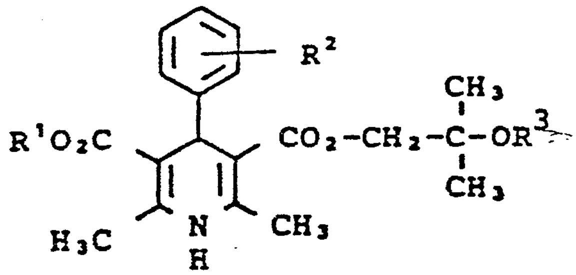

(I)

in welcher

$R^1$ für einen geradkettigen, verzweigten oder cyclischen, gesättigten oder ungesättigten Kohlenwasserstoffrest mit bis zu 6 C-Atomen steht,

$R^2$ für 2-Nitro oder 3-Nitro steht, und

$R^3$ für Wasserstoff oder Acetyl steht,

dadurch gekennzeichnet, daß man

[A] 2-Benzyliden-acetessigester der allgemeinen Formel II

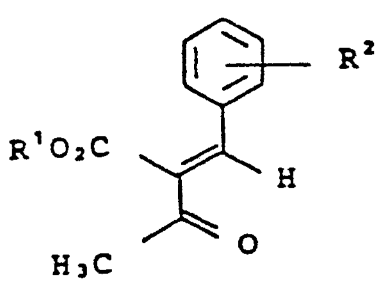

(II)

in welcher

$R^1$ und $R^2$ die oben angegebene Bedeutung haben,

mit 2-Aminocrotonsäure-(2-acetoxy-2-methyl-propyl)ester der Formel III

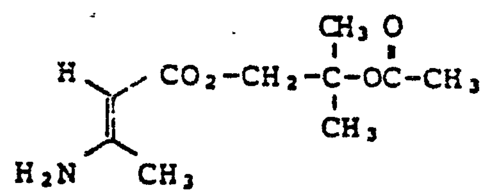

(III)

gegebenenfalls in Gegenwart von Wasser und/oder inerten organischen Lösungsmittel umsetzt,

oder

(B) 2-Benzyliden-acetessigester der allgemeinen Formel II mit Ammoniak und Acetessigsäure-(2-acetoxy-2-methyl-propyl)ester der Formel IV,

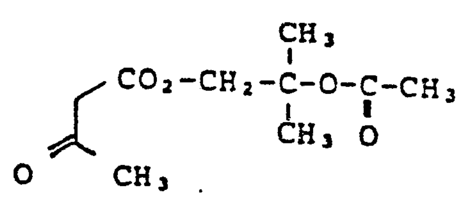

(IV)

gegebenenfalls in Gegenwart von Wasser und/oder inerten organischen Lösungsmitteln umsetzt,

oder

(C) Enamine der allgemeinen Formel V,

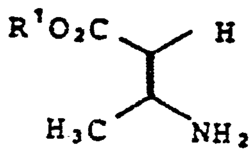

(V)

in welcher

$R^1$ die oben angegebene Bedeutung hat,

mit 2-Benzyliden-acetessigestern der allgemeinen Formel VI,

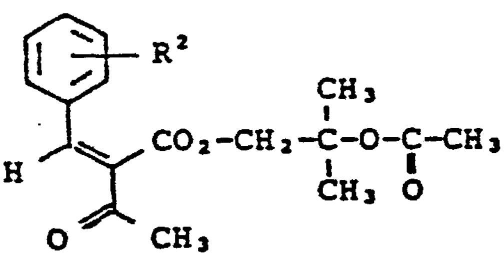

(VI)

in welcher

$R^2$ die oben angegebene Bedeutung hat,

gegebenenfalls in Gegenwart von Wasser und/oder inerten organischen Lösungsmitteln umsetzt,

oder

(D) 2-Benzyliden-acetessigester der allgemeinen Formel VI mit Ammoniak und Acetessigestern der allgemeinen Formel VII,

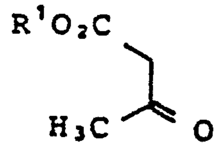

(VII)

in welcher

$R^1$ die oben angegebene Bedeutung hat,

gegebenenfalls in Gegenwart von Wasser und/oder inerten organischen Lösungsmitteln umsetzt,

oder

(E) Enamine der allgemeinen Formel V und Acetessigsäure-(2-acetoxy-2-methyl-propyl)ester der Formel IV mit Aldehyden der allgemeinen Formel VIII,

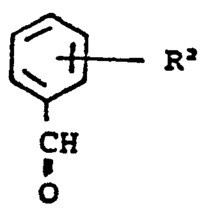

(VIII)

in welcher

$R^2$ die oben angegebene Bedeutung hat,

gegebenenfalls in Gegenwart von Wasser und/oder inerten organischen Lösungsmitteln umsetzt,

oder

(F) 2-Aminocrotonsäure-(2-acetoxy-2-methyl-propyl)ester der Formel III mit Acetessigestern der allgemeinen Formel VII und Aldehyden der allgemeinen Formel VIII gegebenenfalls in Gegenwart von Wasser und/oder inerten organischen Lösungsmitteln umsetzt,

und anschließend die so erhaltenen 1,4-Dihydropyridine der allgemeinen Formel Ib,

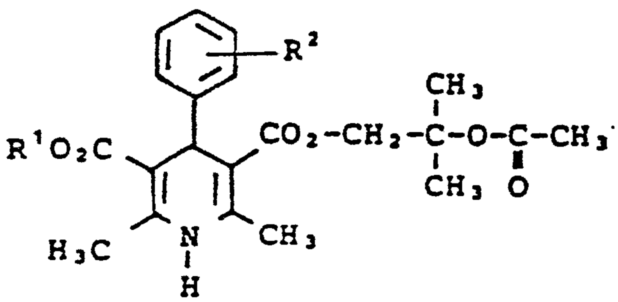

(Ib)

in welcher

$R^1$ und $R^2$ die oben angegebene Bedeutung haben,

unter solvolytischen Bedingungen, bei Temperaturen zwischen 0 und 180°C, gegebenenfalls in Gegenwart von inerten Lösungsmitteln umsetzt.

4. Verfahren gemäß Anspruch 3, dadurch gekennzeichnet, daß man die Umsetzung bei Temperaturen zwischen 20 und 150°C durchführt.

5. Arzneimittel enthaltend mindestens eine Verbindung der allgemeinen Formel I gemäß Anspruch 1.

6. Verfahren zur Herstellung von Arzneimitteln, dadurch gekennzeichnet, daß man Verbindungen der allgemeinen Formel I gemäß Anspruch 1 gegebenenfalls unter Verwendung von inerten Hilfs- und Trägerstoffen in eine geeignete Applikationsform überführt.

7. Verbindungen der allgemeinen Formel I gemäß Anspruch 1 zur Verwendung bei der Bekämpfung von Kreislauferkrankungen.

8. Verwendung von Verbindungen der allgemeinen Formel I gemäß Anspruch 1 bei der Bekämpfung von Erkrankungen.

9. Verwendung von Verbindungen der allgemeinen Formel I gemäß Anspruch 1 bei der Herstellung von kreislaufwirksamen Arzneimitteln.

0199238

Nummer der Anmeldung

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 86105093.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | DE - A1 - 2 847 236 (BAYER)<br>* Ansprüche 1-5 *<br>-- | 1,2,3,4,5,6 | C 07 D 211/90<br>A 61 K 31/44 |
| A | DE - A1 - 3 238 705 (MARUKO)<br>* Zusammenfassung *<br>---- | 1,5,7,9 | |

RECHERCHIERTE SACHGEBIETE (Int. Cl.4)

C 07 D 211/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7,9

Unvollständig recherchierte Patentansprüche: -

Nicht recherchierte Patentansprüche: 8

Grund für die Beschränkung der Recherche:

Verfahren zur therapeutischen Behandlung des menschlichen oder tierischen Körpers Art. 52(4), EPÜ

| Recherchenort | Abschlußdatum der Recherche | Prüfer |
|---|---|---|
| WIEN | 09-07-1986 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82